(19) Europäisches Patentamt
European Patent Office
Office européen des brevets

(11) EP 1 524 281 A2

(12) **DEMANDE DE BREVET EUROPEEN**

(43) Date de publication:
**20.04.2005 Bulletin 2005/16**

(21) Numéro de dépôt: **05001315.0**

(22) Date de dépôt: **28.02.1997**

(51) Int Cl.[7]: **C08G 18/08**, C08G 18/38, C08G 18/79, C08G 18/80, C09D 175/04

(84) Etats contractants désignés:
**AT BE CH DE DK ES FI FR GB GR IE IT LI LU MC NL PT SE**

(30) Priorité: **29.02.1996 FR 9602710**

(62) Numéro(s) de document de la (des) demande(s) initiale(s) en application de l'article 76 CBE:
**97907149.5 / 0 815 153**

(71) Demandeur: **Rhodia Chimie**
**92100 Boulogne Billancourt (FR)**

(72) Inventeurs:
- **Nabavi, Minou**
  **75014 Paris (FR)**
- **Jeannette, Thierry**
  **92380 Garches (FR)**
- **Lyothier, Arnaud**
  **93300 Aubervilliers (FR)**
- **Bernard, Jean-Marie**
  **Saint Laurent d'Agny 69440 Mornant (FR)**

(74) Mandataire: **Colombet, Alain André et al**
**Cabinet Lavoix,**
**2, Place d'Estienne d'Orves**
**75441 Paris Cedex 09 (FR)**

Remarques:
Cette demande a été déposée le 24 - 01 - 2005 comme demande divisionnaire de la demande mentionnée sous le code INID 62.

(54) **Compositions à base d'isocyanate, leur procédé d'utilisation leur utilisation pour réaliser des revêtements et revêtements ainsi obtenus**

(57) La présente invention a pour objet des compositions pour addition successive ou simultanée comprenant :

- un composé (poly)isocyanate non masqué ou partiellement masqué, et
- un agent tensioactif contenant au moins un composé comportant une fonction anionique et avantageusement un fragment de chaîne polyéthylèneglycol d'au moins 1, unité éthylèneoxyle.

La présente invention a également pour objet les compositions définies ci-dessus et qui comprennent en outre un composé à hydrogène réactif.

Applications dans le domaine des revêtements, peintures ou vernis.

EP 1 524 281 A2

## Description

La présente invention a pour objet des composés et des compositions à base d'isocyanate (qui peuvent être partiellement masqués, mais cela n'est pas la mise en oeuvre préférée). Elle vise également leur procédé d'utilisation, leur utilisation pour réaliser des revêtements et revêtements ainsi obtenus. Elle concerne plus particulièrement les compositions (auto)dispersibles en phase aqueuse.

Pour mieux comprendre l'invention il a paru opportun de faire les rappels suivants.

Dans la présente description, les caractéristiques de granulométrie font souvent référence à des notations du type $d_n$ où n est un nombre de 1 à 99, cette notation est bien connue dans de nombreux domaines techniques, mais est un peu plus rare en chimie, aussi peut-il être utile d'en rappeler la signification. Cette notation représente la taille de particule telle que n % (en poids, ou plus exactement en masse, puisque le poids n'est pas une quantité de matière mais une force) des particules soit inférieur ou égal à ladite taille.

Dans la suite de la description, on utilisera l'indice de polydispersité, lequel est défini comme

$$I = (d_{90}-d_{10})/d_{50}$$

Dans l'activité des peintures et des vernis, on utilise largement les diisocyanates notamment les alcoylènes diisocyanates (par exemple ceux vendus sous la marque Tolonate) et leurs dérivés de type biuret ou leurs trimères.

Toutefois, deux problèmes restent à ce jour incomplètement résolus, à savoir :

- l'utilisation de solvant organique, dont la présence est réputée toxique et néfaste pour l'environnement ;
- la nécessité de mettre sur le marché des produits non volatils qui a conduit à alourdir les molécules et ce en oligomérisant les diisocyanates ; cette solution n'est pas entièrement satisfaisante car elle utilise une fonction élaborée, donc chère, pour résoudre le problème.

Bien entendu, ces problèmes doivent être résolus en respectant les contraintes intrinsèques aux revêtements.

Pour réaliser des films de peintures ou de vernis, on mélange d'une part une dispersion, ou une émulsion, contenant l'isocyanate qui peut être bloqué et d'autre part une dispersion ou une solution de polyol.

Le mélange des dispersions pouvant également contenir des pigments, et notamment du bioxyde de titane, dont la dispersion est améliorée par la présente invention, et des charges est alors déposé sur un support sous forme d'un film à l'aide des techniques classiques de mise en oeuvre des peintures industrielles. Lorsque la préparation contient des isocyanates bloqués, l'ensemble film plus support est cuit à une température suffisante pour assurer le déblocage des fonctions isocyanates et la condensation de celles-ci avec des groupements hydroxyles des particules de polyol. Il convient toutefois de rappeler que les produits masqués ou bloqués présentent un prix de revient significativement plus élevé que celui des produits non masqués.

L'utilisation de solvants organiques est de plus en plus souvent critiquée par les autorités en charge de la sécurité du travail, car ces solvants, ou du moins certains d'être eux, sont réputés toxiques ou chronotoxiques. C'est la raison pour laquelle on essaye de développer de plus en plus des techniques qui se substituent aux techniques en milieu solvant pour pallier les inconvénients afférents aux solvants.

Une des solutions les plus souvent utilisées réside dans l'utilisation d'émulsions ou de dispersions dans l'eau. En raison de la réactivité de l'eau avec les isocyanates, cette solution est surtout utilisée pour les isocyanates masqués.

Pour ne pas tomber de Charybde en Scylla, un écueil majeur est à éviter, à savoir détériorer une ou plusieurs des qualités essentielles des revêtements [caractère lisse (éviter la "peau d'orange"), dureté, résistance aux solvants,.....]. en particulier, il faut craindre une mauvaise adhésion du revêtement sur son support. En effet beaucoup de tensioactifs sont réputés altérer la solidité du lien entre le revêtement et son support et sont connus et utilisés pour défavoriser l'accrochage entre un polymère et un support.(confère par exemple DE-OS 3.108.537).

Lorsque l'on utilise des isocyanates non ou incomplètement masqués, la durée pendant laquelle il sont utilisables reste inférieur à quelques heures, En général une ou deux heures.

Aussi est-il important de ne pas rencontrer de difficulté lors de la mise en émulsion ou en dispersion des isocyanates.

C'est pourquoi un des buts de la présente invention est de fournir une composition qui permette par mélange dans l'eau ou plus précisément dans une phase aqueuse, d'obtenir une émulsion sans qu'il soit pour cela nécessaire d'utiliser des techniques et/ou des installions spécifiques.

Un autre but de la présente invention est de fournir une composition du type précédent qui ne perturbe pas les opérations de revêtement.

Un autre but de la présente invention est de fournir une composition du type précédent dont la teneur en solvant soit inférieur à 1/5, avantageusement à 1/10, en masse de ladite composition. Bien sûr il est préférable qu'il

n'y en ait le moins possible, et même pas du tout.

Ces buts et d'autres qui apparaîtront par la suite sont atteints au moyen d'une composition à base d'isocyanate (s), avantageusement non masqué(s), où elle comporte au moins un composé comportant une fonction anionique et avantageusement un fragment de chaîne polyéthylène glycol d'au moins 1, de préférence d'au moins 5 unités éthylènyloxyles

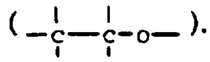

ainsi la présente invention vise pour addition successive ou simultanée, une composition comportant notamment :

→ une sous composition vecteur de fonctions isocyanate dont les caractéristiques préférées seront précisée ultérieurement et

→ un agent tensioactif contenant au moins un composé comportant une fonction anionique et avantageusement un fragment de chaîne polyéthylène glycol d'au moins 1, de préférence d'au moins 5 unités éthylènyloxyles

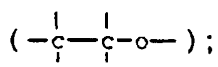

⇨ optionnellement une phase aqueuse.

Selon la présente invention, ledit composé peut-être utilisé seul, ou en mélange avec un ou plusieurs agents tensioactifs. Ces derniers peuvent être des agents répondant aussi à l'obligation ci-dessus de comporter une fonction anionique et avantageusement un fragment de chaîne polyéthylène glycol de préférence d'au moins 5 unités éthylènyloxyles.

Ces éventuels agents tensioactifs peuvent également être choisis parmi d'autres composés ioniques [notamment sulfate ou phosphate d'aryle(s) et/ou d'alcoyle(s) (bien entendu aryle englobe notamment les alcoylaryles et alcoyle englobe notamment les aralcoyles), les aryl- ou alcoyl--phosphonate, phosphinate, sulfonate, sel d'acide gras et/ou zwitterionique] et parmi les composés non-ioniques ceux bloqués en bout de chaîne ou non. (toutefois les composés non ioniques présentant des fonctions alcooliques sur au moins l'une des chaînes semblent avoir un effet légèrement défavorable sur l'(auto)émulsion même si ils ont un effet favorable sur d'autres aspects de la composition pour peinture ; compte tenu de cela il est préférable que la teneur en ce type de composé représentent au plus un tiers, avantageusement au plus un cinquième, de préférence au plus un dixième en masse desdits composés anioniques selon l'invention)

Avantageusement ledit composé comporte une partie hydrophile formée de ladite fonction anionique, dudit (éventuel) fragment de chaîne polyéthylène glycol et d'une partie lipophile à base d'un radical hydrocarboné.

Ladite partie lipophile est en général choisie parmi les groupes alcoyles [dans la présente description ALco-***yle*** est pris dans son sens étymologique de reste hydrocarboné d'un **ALCO-*ol*** après ignorance de la fonction alcool (ou ol)] ;et aryles. Lorsque le nombre de fonction éthylène glycol est au plus égale à 5, les alcoyles simples sont avantageusement ramifiés, avantageusement de $C_8$ à $C_{12}$, les aralcoyles $C_{12}$ à $C_{16}$, les alcoylaryles de $C_{10}$ à $C_{14}$ et les aryles simples sont des $C_{10}$ à $C_{16}$ Sinon la partie lipophile peut varier largement surtout lorsque le nombre de motifs éthylène glycol est au dessus de 10, elle peut ainsi constitué un radical hydrocarboné d'au moins 1, avantageusement d'au moins 3 et comportant au plus 25 avantageusement au plus 20 atomes de carbone.

Avantageusement ledit composé répond à la formule I suivante.

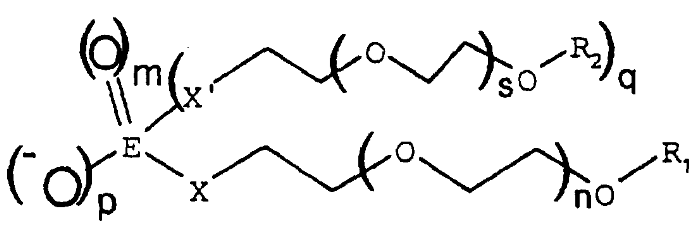

| | |
|---|---|
| Où q | représente zéro ou 1 ; |
| où p | représente un entier entre 1 et 2 (intervalles fermés c'est à dire comprenant les bornes) ; |

où m représente zéro ou un entier entre 1 et 2 (intervalles fermés c'est à dire comprenant les bornes) ;
où X et X', semblables ou différents, représente un bras comportant au plus deux chaînons carbonés ;
où s est zéro ou un entier choisi entre 1 et 30 avantageusement entre 5 et 25, de préférence entre 9 et 20 (intervalles fermés c'est à dire comprenant les bornes) ;
où n est zéro ou un entier choisi entre 1 et 30 avantageusement entre 5 et 25, de préférence entre 9 et 20 (intervalles fermés c'est à dire comprenant les bornes) ;
où E est un élément choisi parmi le carbone et les éléments métalloïdes de rang atomique au moins égal à celui du phosphore et appartenant à la colonne VB ou aux chalcogènes de rang atomique au moins égal à celui du soufre ;
où $R_1$ et $R_2$, semblables ou différents, représentent un radical hydrocarboné, avantageusement choisi parmi les aryles et les alcoyles éventuellement substitués.

Quoique cela ne fasse pas partie des composés préférés, il convient de noter que s et/ou n peuvent être égaux à zéro, avec la condition que E est phosphore et que lorsque s et n sont égaux à zéro, respectivement R1 et/ou R2 sont alcoyles de $C_8$ à $C_{12}$, avantageusement ramifié, ou un aralcoyle de $C_{12}$ à $C_{16}$ ou un alcoyle aryle de $C_{10}$ à $C_{14}$.
L'un des radicaux divalents X et X' peut aussi être un radical de type ($[EO_m\ (O-)_p]$) de manière à former des pyroacides comme les diesters symétrique ou non de l'acide pyrophosphorique .
Le nombre total de carbone des composés anioniques visés par la présente invention est avantageusement d'au plus environ 100, de préférence au plus environ 50.
Les radicaux divalent X et éventuellement X' sont avantageusement choisis parmi les radicaux divalents constitués par (la partie gauche de la formule étant lié au premier E) :

⇨ quand E est P, un des X ou X' peut être $O-P(O)(O^-)-X''-$ ;
⇨ quand E est P, un des X ou X' peut être $-O-(R_{10}-O)P(O)-X''-$ ; ($R_{10}$ étant défini ci après)(X" représentant un oxygène ou une simple liaison) ;

→ une liaison directe entre E et le premier éthylène dudit fragment de chaîne polyéthylène glycol ;
→ les méthylènes éventuellement substitués et dans ce cas avantageusement fonctionnalisés en parti ;
→ les bras de structure -Y- et de structure -D-Y-, -Y-D-, -Y-D-Y'

où Y représente un chalcogène (avantageusement choisi parmi les plus légers à savoir le soufre et surtout l'oxygène), les éléments métalloïdes des rangs atomique au plus égal à celui du phosphore et appartenant à la colonne VB sous la forme de dérivés d'amines ou de phosphines tertiaires (le radical assurant le caractère tertiaire étant avantageusement d'au plus 4 carbones, de préférence d'au plus 2 carbones) ;
où D représente un alcoylène éventuellement substitué, y compris fonctionnalisé, D étant avantageusement éthylène ou méthylène, de préférence éthylène dans les structures -D-Y-et surtout -Y-D-Y', et méthylène dans les structures -Y-D-,

Ainsi E représente un atome choisi parmi les atomes de carbone (avantageusement dans ce cas m =1 et p = 1, le prototype de ce type de composé est un acide alcool [Par exemple acide lactique ou glycolique] polyéthoxylé) les atomes donnant les pnictures (éléments de la colonne VB)(avantageusement dans ce cas m = 1 ou 0 et p = 1 ou 2), les atomes de chalcogène de rang supérieur à l'oxygène (avantageusement dans ce cas m =1 ou 2 et p = 1 et q =0).
Ainsi dans le cas où E est chalcogène la formule I se simplifie avantageusement en :

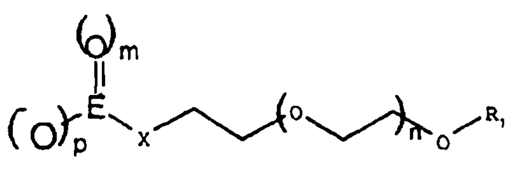

Avantageusement E représente le carbone et surtout le phosphore ou le soufre, de préférence le phosphore :

dans ce dernier cas la formule (I) devient la formule (II) :

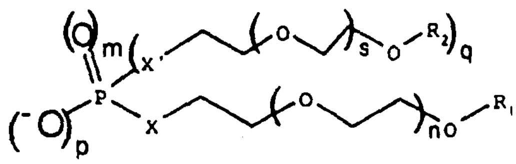

Avec lorsque q est zéro :

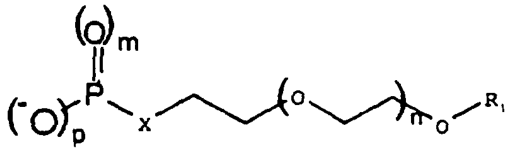

- Où p représente zéro ou un entier entre 1 et 2 (intervalles fermés c'est à dire comprenant les bornes) ;
- où m représente zéro ou un entier entre 1 et 2 (intervalles fermés c'est à dire comprenant les bornes) ;
- où la somme p + m + q est au plus égale à trois ;
- où la somme 1 + p + 2m + q est égale à trois ou à cinq ;
- où X et X', semblables ou différents, représentent un bras comportant au plus deux chaînons carbonés ;
- où n et s, semblables ou différents, représentent un entier choisi entre 5 et 30 avantageusement entre 5 et 25, de préférence entre 9 et 20 (intervalles fermés c'est à dire comprenant les bornes) ; où $R_1$ et $R_2$, semblables ou différents, représentent un radical hydrocarboné, avantageusement choisi parmi les aryles et les alcoyles éventuellement substitués notamment par atome d'halogène notamment fluor.

La classification périodique des éléments utilisée dans la présente demande est celle du supplément au Bulletin de la Société Chimique de France, janvier 1966, n° 1.
La fonctionnalisation éventuelle des alcoylènes et notamment méthylènes (X et X') est faite par des fonctions hydrophiles (amines tertiaires et autres fonctions anioniques y compris celles qui sont décrites ci-dessus [$EO_m$ $(O-)_p$]).
Le contre-cation est avantageusement monovalents et est choisi parmi les cations minéraux et les cations organiques avantageusement non nucléophile et par voie de conséquence de nature quaternaire ou tertiaire (notamment oniums de colonne V tel que phosphonium, ammoniums, voire de colonne VI tel que sulfonium,.....) et leurs mélanges, le plus souvent ammoniums, en général issus d'une amine, avantageusement tertiaire. Avantageusement, on évite que 'le cation organique présente un hydrogène réactif avec la fonction isocyanate. D'où la préférence vis-à-vis des amines tertiaires.
Les cations minéraux peuvent être séquestrés par des agents de transfert de phases comme les éthers couronnes
Le pKa des cations (organique [ammonium.....] ou minéraux) est avantageusement compris entre 8 et 12.

. Les cations et notamment les amines correspondant aux ammoniums ne présentent avantageusement pas de propriété tensioactive mais il est souhaitable qu'elles présentent une bonne solubilité en tout cas suffisante pour assurer celle desdits composés comportant une fonction anionique et avantageusement un fragment de chaîne polyéthylène glycol, en phase aqueuse et ce à la concentration d'emploi. Les amines tertiaires présentant au plus 12 atomes, avantageusement au plus 10 atomes, de préférence au plus 8 atomes de carbone par fonction "onium" (rappelons qu'il est préféré qu'il n'y en ait qu'une par molécule) sont préférées. Les amines peuvent comporter d'autre fonction et notamment les fonctions correspondant aux fonctions des acides aminés et des fonctions éther cyclique comme la N-méthylmorpholine, ou non. Ces autres fonctions sont avantageusement sous une forme qui ne réagisse pas avec les fonctions isocyanates et ne altèrent significativement pas la solubilité en phase aqueuse.

Il est très souhaitable que les composés anioniques selon la présente invention soient sous une forme neutralisée telle que le pH qu'elle induit lors d'une dissolution ou d'une mise en contact dans l'eau soit compris au moins égal à 3, avantageusement à 4, de préférence à 5 et au plus égal à 12, avantageusement à 11, de préférence à 10.
Lorsque E est phosphore il est souhaitable d'utiliser des mélange de monoester et de diester dans un rapport molaire compris entre 1/10 et 10, avantageusement entre 1/4 et 4. De tel mélange peuvent en outre comporter de 1 % jusqu'à environ 20 % (il est toutefois préférable que cela ne dépasse pas environ 10 %) en masse d'acide phosphorique (qui sera avantageusement salifié au moins en partie de manière à être dans les zones de pH préconisée). et de 0 à 5 % d'esters de l'acide pyrophosphorique.

Le rapport massique entre les composés tensioactifs (y compris ledit composé comportant une fonction anionique et avantageusement un fragment de chaîne polyéthylène glycol) et les isocyanates est très préférablement compris entre 4 et environ 10 %. les zones préconisées seront explicitée ultérieurement.

La composition peut comporter en outre un catalyseur avantageusement latent (libérable par action des agents extérieurs, par exemple rayonnement visible ou U.V., oxygène).

La composition d'isocyanate selon l'invention peut comporter après mise en dispersion ou émulsion dans une phase aqueuse, une teneur en eau de 10 à 70%. L'émulsion est une émulsion huile dans eau.

Toutefois, au cours de l'étude qui a mené à la présente invention, en particulier dans le cas des isocyanates aliphatiques (c'est-à-dire reliés au squelette hydrocarboné (c'est-à-dire contenant à la fois de l'hydrogène et du carbone) par l'intermédiaire d'un carbone saturé ($sp^3$) il a été montré qu'il avait un risque d'emballement de diverses réactions lorsque certaines proportions d'eau était atteinte, Aussi est-il avisé d'éviter des compositions où le rapport massique entre d'une part la quantité d'eau dans la phase aqueuse et d'autre part la somme de l'isocyanate et du tensioactif selon l'invention, est compris entre $10^{-2}$ et un demi. Si l'on désire plus de sûreté on évitera les rapports compris entre $10^{-3}$ et 1.

Les émulsions obtenues présentent pour la partie isocyanate des $d_{50}$ au moins égal à 0,1 micromètre, plus souvent à 0,5 micromètre et elles présentent un $d_{50}$, préférentiellement un $d_{80}$, avantageusement ≤ (au plus égal à) 5 micromètres, de préférence à 3 micromètres.

La phase aqueuse de l'émulsion, sert en général de vecteur des co-réactifs polycondensables avec les fonctions isocyanates et comporte alors des composés présentant des fonctions (avantageusement au plus 4, de préférence au plus 3 fonctions cf. ce qui est expliqué ci après pour les polyol qui général mutatis mutandis à toutes les fonctionnalité de ce type) à hydrogènes réactifs, en général un ou plusieurs polyols.

Ce polyol est un polymère qui contient au moins 2 groupements hydroxyles (phénol ou alcool) ayant avantageusement un taux d'hydroxyle entre 0,5 et 5, avantageusement entre 1 et 3 % (en masse). sauf dans le cas des latex qui sera rappelé ci-après, il comporte avantageusement au plus 4, de préférence au plus 3 fonctions alcools primaires (mais le plus souvent deux car la réticulation proprement dite [provoquée par une fonctionnalité statistiquement supérieure à deux (valeur fractionnaire possible)] est en général engendrée par les polyisocyanates). Mais il peut comporter en outre des fonctions alcooliques secondaire ou tertiaire (en général au plus environ 10, avantageusement au plus 5, plus souvent au plus deux) qui, en général, ne réagissent pas ou ne réagissent qu'après les primaires, et ce dans l'ordre primaire, secondaire et tertiaire.

Les polyoses ou polyosides (amidon, cellulose, gommes (guar, caroube, xanthane,....) diverses....) surtout sous forme solide sont à éviter. Sous la forme d'agent texturant, et dans la mesure où cela ne gène pas la mise en émulsion et la stabilité de cette dernière, il peuvent toutefois être utilisés pour donner des propriétés particulières (Par exemple thyxotropie....). Le squelette du polymère peut être de nature chimique diverse notamment acrylique, polyester, alkyde, polyuréthanne, voire amide, y compris urée.

Le polyol peut comporter des groupements anioniques, notamment carboxyliques ou sulfoniques ou ne pas comporter de groupement ionique.

Dans le cadre de la présente invention, il a été montré que la présente de fonction carboxylate anionique ($-CO_2^-$) augmentait significativement la cinétique de séchage ce qui est particulièrement intéressant pour obtenir un "hors poussière" rapide, notamment lorsque l'on opère à l'extérieur. Un effet significatif peut être noté pour un rapport d'au moins d'une fonction carboxylique pour environ 20 fonctions à hydrogène réactif [fonction alcool ou phénol], avantageusement pour un rapport d'un pour environ 10, de préférence pour un rapport d'un pour environ 5 ; il est toutefois souhaitable que ce rapport soit au plus égale à une fonction pour une fonction, de préférence d'un fonction carboxylique pour deux fonctions ol. Les contre cations des carboxylate répondent avantageusement au mêmes préférence que celles exprimée pour les contre cation du composé selon la présente invention.

Le polyol peut déjà être en milieu aqueux ou hydrosoluble ou hydrodispersable.

Il peut s'agir d'une solution aqueuse (qui peut notamment être obtenue après neutralisation des groupements ioniques) ou d'une émulsion du polymère dans l'eau ou d'une dispersion de type latex.

Il semble possible de disperser un polyisocyanate standard dans un polyol hydrosoluble dans certaines conditions de formulation (notamment avec un rapport pigment sur liant de la peinture adapté). Mais l'utilisation de polyisocyanates standards avec des polyols hydrodispersés (types émulsion de résine ou latex), pose souvent des problèmes d'incompatibilité (floculation, apparition de plusieurs phases...). Un des nombreux avantages de la préparation selon l'invention est qu'elle offre une grande liberté de choix pour la formulation (forme physique du polyol, rapport pigment sur liant, facilité d'incorporation dans les milieux aqueux).

D'autre part il a été constaté au travers des valeurs d'usages des revêtements (notamment résistance chimique et dureté) que la réticulation des films était beaucoup plus importante quand le polyol utilisé était carboxylé.

En particulier on peut utiliser avantageusement les latex, notamment les nanolatex (c'est-à-dire latex dont la

taille particulaire est nanométrique [plus précisément dont le $d_{50}$ est au plus égal à environ 100 nanomètres])

Ainsi, selon une des mises en oeuvre particulièrement avantageuses de la présente invention, le polyol est avantageusement un latex de taille nanométrique présentant les caractéristiques suivantes :

$d_{50}$ compris entre 15 et 60 nm, avantageusement entre 20 et 40 nm
fonction carboxylate de 0,5 à 5 % en masse
fonction ol : entre 1 et 4 % avantageusement entre 2 et 3 %
taux de solide : entre 25 et 40 %
un $d_{80}$ inférieur à 1 micromètre

En outre les latex, surtout quand leur point de transition vitreuse est inférieur à 0°C, avantageusement à -10°C, de préférence à -20°C, permettent l'obtention même avec des isocyanates aromatiques de bonne qualité de résistance aux intempéries et notamment aux variations de température.

Le rapport molaire entre les fonctions isocyanates libres et les fonctions hydroxyle est compris entre 0,5 et 2,5, avantageusement entre 0,8 et 1,6, avantageusement entre 1 et 1,4.

Les latex (non fonctionnalisés en isocyanate éventuellement masqués) décrits dans la demande de brevet d'invention français déposé le 28 avril 1995 N° 95/05123 et dans la demande réflexe de brevet Européen N°EP 0.739.961 donnent de forts bons résultats.

Ainsi avantageusement les particules de latex présentent une teneur en fonction acide (avantageusement carboxylique), accessible comprise entre 0,2 et 1,2 milliéquivalent/gramme de matière solide et qu'elles présentent une teneur en fonction alcoolique accessible comprise entre 0,3 et 1,5 milliéquivalent/gramme.

Ainsi, comme indiqué dans ce document sont préférés les latex constitués de particules porteuses de fonction (s) selon l'invention, sont hydrophobes et possèdent avantageusement une taille ($d_{90}$) généralement comprise entre 0,01 micromètre et 10 micromètres et de préférence au plus égale à 5 micromètres, voire à 2 micromètres. Elles sont calibrées, monodispers(é)es et présentes dans le latex à raison d'une quantité variant entre 0,2 à 65 % en poids du poids total du latex.

La masse moléculaire moyenne en poids ($M_w$ déterminée de préférence par chromatographie de perméation de gel, connue sous l'acronyme anglo-saxon de "GPC") des polymères constitutifs des particules de la population A (latex à fonction ol jouant le rôle de polyol) est avantageusement comprise entre $5.10^4$ et $5.10^6$, de préférence 0,8 $10^5$ et $2.10^6$.

Les fonctions alcooliques ou les fonctions acides, de préférence carboxyliques peuvent être également obtenues par hydrolyse de fonctions alcoologènes (ester, éther, halogénure...) ou de fonctions acidogènes (ester, anhydride, chlorure d'acide, amide, nitrile...).

La répartition entre les différents types de motifs répond avantageusement aux règles suivantes :

La teneur en motif issu du monomère constitué par ledit alcool libre présentant une fonction éthylénique activée, et rapportée à la totalité des motifs de toute sorte, est avantageusement comprise entre 3 et 15 %, de préférence entre 4 et 10 % (mole, ou équivalent).

Selon un mode avantageux de la présente invention, le motif est issu d'un ester, d'un acide alpha éthylénique, avec un diol dont une des fonctions alcool reste non estérifiée. Ledit diol est avantageusement un diol oméga/oméga prime, avantageusement choisi parmi le butanediol-1,4, le propane diol-1,3 et le glycol.

Il est souhaitable que ledit acide alpha éthylénique soit un acide acrylique éventuellement substitué.

Selon un mode préféré de la présente invention, la teneur en motif issue d'un acide carboxylique libre (ou sous forme d'un de ses sels), et rapportée à la totalité des motifs de toute sorte, est comprise entre 2 et 10 % (mole).

Pour des raisons économiques, il est souvent avantageux que ledit acide libre soit un acide acrylique éventuellement monosubstitué ou un de ses sels.

Les particules issues de la présente invention peuvent être constituées de deux polymères distincts, le premier constituant le coeur et le second constituant la périphérie. Ce type de particule peut être obtenu par épipolymérisation [où une semence de latex est recouverte par polymérisation superficielle (épipolymérisation, parfois désignée par surpolymérisation)] d'un polymère distinct. Le coeur est parfois appelé germe par analogie au phénomène de cristallisation. Dans ce cas là, seul le deuxième polymère, c'est-à-dire le polymère superficiel, répond aux contraintes de concentration aux différentes fonctions selon la présente invention.

Le rapport massique entre les isocyanates à mettre en suspension et ledit composés comportant une fonction anionique et avantageusement un fragment de chaîne polyéthylène glycol est le plus souvent au plus égal à 1/3 avantageusement au plus égal à environ 20%, de préférence à environ 10 %. (dans la présente description le terme "environ" est employé pour mettre en exergue le fait que, lorsque le, ou les, chiffres les plus à droite d'un nombre sont des zéros, ces zéros sont des zéros de position et non des chiffres significatifs, sauf bien entendu s'il en est précisé autrement).

Le rapport massique entre les isocyanates à mettre en suspension et ledit composé comportant une fonction anionique et avantageusement un fragment de chaîne polyéthylène glycol est avantageusement supérieur à 1 %, de

préférence à 2 %.

Il est également souhaitable que la quantité dudit ou desdits composés comportant une fonction anionique et avantageusement un fragment de chaîne polyéthylène glycol corresponde à une valeur comprise entre $10^{-2}$ et 1, avantageusement entre 5.10-2 et 0,5 atome de E par litre

Ainsi le rapport massique entre les isocyanates à mettre en suspension et ledit composé comportant une fonction anionique et avantageusement un fragment de chaîne polyéthylène glycol est avantageusement au moins égal à 2 %, de préférence à 4 %, et au plus égal à environ 20 %, de préférence à environ 10 %, ainsi ce rapport massique est avantageusement compris entre et environ 20 %, de préférence entre 4 et environ 10 %.

Selon une mise en oeuvre particulièrement avantageuse de la présente invention, après mise en dispersion ou émulsion, la somme des constituants du liant (c'est-à-dire des teneurs en masse du, ou des, isocyanates, des émulsifiants et des polyols), dans l'eau varie de 30 à 70 % par rapport à la totalité de la composition.

Les isocyanates visés par l'invention comporte notamment les composés détaillés ci après.

Ces composés peuvent avantageusement contenir les structures courantes dans ce domaine par exemple les prépolymères issus de la condensation de polyol (Par exemple triméthylol propane) en général triol (avantageusement primaire voire plus loin sur la définition des polyols) et surtout les plus courantes, à savoir celles de type isocyanurate, encore appelée trimère, des structures urétidine dione, encore appelée dimère, des structures biuret ou allophanate ou une combinaison de ce type de structures sur une seule molécule ou en mélange.

Si l'on désire abaisser fortement la teneur en solvant de la composition, notamment lorsqu'elle sous forme d'émulsion, il est préférable d'utiliser des mélanges de ce type naturellement (c'est-à-dire sans adjonction de solvant) à basse viscosité. Les composés présentant cette propriété sont surtout les dérivés (type isocyanurate, encore appelée trimère, des structures urétidine dione, encore appelée dimère, des structures biuret ou allophanate ou une combinaison de ce type de structures sur une seule molécule ou en mélange) en partie et/ou en totalité des isocyanates aliphatiques dont les fonctions isocyanate sont reliées au squelette par l'intermédiaire de fragments éthylène (Par exemple des polyméthylènediisocyanates, notamment l'hexaméthylènediisocyanate et ceux des arylènedialcoylènediisocyanates dont la fonction isocyanate est distante des noyaux aromatique d'au moins deux carbones tels que ($OCN-[CH_2]_t-\Phi-[CH_2]_u-NCO$) avec t et u supérieur à 1). Ces composés ou mélanges ont avantageusement un viscosité au plus égale à environ 3000 centipoises (ou millipascal.seconde), de préférence à environ 1500 centipoises (ou millipascal.seconde).

Lorsque ces valeurs ne sont pas atteintes Il est alors souvent utile d'amener le mélange à ces valeurs de viscosité par l'adjonction d'une quantité minimale de solvant(s) approprié(s).Ainsi que déjà mentionné ci-dessus, les isocyanates concernés peuvent être des mono-, di- voire poly-isocyanates. Avantageusement, ces dérivés peuvent contenir des structures de type isocyanurate, encore appelée trimère, des structures urétidine dione, encore appelée dimère, des structures biuret ou allophanate ou une combinaison de ce type de structures sur une seule molécule ou en mélange.

Les isocyanates monomères peuvent être :

⇨ aliphatiques, y compris cycloaliphatiques et arylaliphatiques, tels que :

- comme aliphatique simples, les potyméthytènediisocyanates et notamment l'hexaméthylène diisocyanate :
- comme aliphatique partiellement "néopentylique" partiellement cyclique (cycloaliphatique) l'isophorone diisocyanate (IPDI) ;
- comme aliphatique cyclique (cycloaliphatique) diisocyanate ceux dérivés du norbornane ;
- les arytènediatcoytènediisocyanates (tel que $OCN-CH_2-\Phi-CH_2-NCO$ dont une partie ne présente pas de différence essentielle d'avec les aliphatiques à savoir ceux dont la fonction isocyanate est distante des noyaux aromatique d'au moins deux carbones tels que ($OCN-[CH_2]_t-\Phi-[CH_2]_u-NCO$) avec t et u supérieur à 1 ;

⇨ ou encore aromatiques tels que le toluylène diisocyanate.

Les polyisocyanates préférés visés par la technique de l'invention sont ceux dans lesquels au moins une, avantageusement deux, de préférence trois des conditions ci après sont remplies :

- Au moins une, avantageusement au moins deux, des fonctions NCO sont reliées à un squelette hydrocarboné, par l'intermédiaire d'un carbone saturé ($sp^3$), de préférence avec au moins une, plus préférentiellement au moins deux des sous conditions ci- après :.

    - Au moins un, avantageusement deux, desdits carbones saturés ($sp^3$) est porteur d'au moins un, avantageusement deux, hydrogène(s),(en d'autre terme il a été trouvé que l'on obtenait de meilleurs résultats lorsque le carbone porteur de la fonction isocyanate était porteur d'un hydrogène, de préférence de deux hydrogènes) ;

- au moins un, avantageusement deux, desdits carbones saturés ($sp^3$) sont eux-mêmes portés par un carbone, avantageusement aliphatique (c'est-à-dire d'hybridation $sp^3$), lui-même porteur d'au moins un, avantageusement deux, hydrogène(s) ; en d'autre terme, il a été trouvé que l'on obtenait de meilleurs résultats lorsque le carbone porteur de la fonction isocyanate n'était en position dite "néopentylique" ;

  - Tous les carbones par l'intermédiaire desquels les fonctions isocyanates sont reliées au squelette hydrocarboné, sont des carbones saturés ($sp^3$), lesquels sont avantageusement en partie, de préférence en totalité, porteurs d'un hydrogène, de préférence de deux hydrogènes ; en outre il est avantageux que lesdits carbones saturés ($sp^3$) soient au moins en partie (avantageusement un tiers, de préférence deux tiers), préférablement en totalité, eux-mêmes portés par un carbone, avantageusement aliphatique (c'est-à-dire d'hybridation $sp^3$), lui-même porteur d'au moins un, avantageusement deux, hydrogène(s) ; en d'autre terme, il a été trouvé que l'on obtenait de meilleurs résultats lorsque le carbone porteur de la fonction isocyanate n'était en position dite "néopentytique' ;

- Sont en particulier bien adaptés ceux qui présentent au moins en partie un squelette isocyanurique ou biuret (que ce squelette soit issu d'un seul ou de plusieurs monomères, voir ci-dessous) et plus précisément des structures de type isocyanurate, encore appelée trimère, des structures urétidinedione, encore appelée dimère, des structures biuret ou allophanate ou une combinaison de ce type de structures sur une seule molécule ou en mélange.

Lorsque les polyisocyanates sont relativement lourds c'est à dire qu'ils comportent au moins 4 fonctions isocyanates, les deux premières conditions deviennent :

- Au moins un tiers, avantageusement deux tiers, des fonctions NCO sont reliées à un squelette hydrocarboné, par l'intermédiaire d'un carbone saturé ($sp^3$) ; .

  - Au moins un tiers, avantageusement deux tiers, desdits carbones saturés ($sp^3$) est porteur d'au moins un, avantageusement deux, hydrogène(s),(en d'autre terme il a été trouvé que l'on obtenait de meilleurs résultats lorsque le carbone porteur de la fonction isocyanate était porteur d'un hydrogène, de préférence de deux hydrogènes.; En outre il est avantageux que lesdits carbones saturés ($sp^3$) soient au moins en partie (avantageusement un tiers, de préférence deux tiers), préférablement en totalité, eux-mêmes portés par un carbone, avantageusement aliphatique (c'est-à-dire d'hybridation $sp^3$), lui-même porteur d'au moins un, avantageusement deux, hydrogène(s) ; en d'autre terme, il a été trouvé que l'on obtenait de meilleurs résultats lorsque le carbone porteur de la fonction isocyanate n'était en position dite 'néopentylique" ;

Les isocyanates notamment aliphatiques réagissent avec certains des composés anioniques visés par l'invention il réagissent avec l'hydroxyle des fonctions acides non ou mal neutralisées. ces composé sont également visé par la présente invention.

En particulier dans le cas des phosphates (m = 1) ils réagissent pour donner des composés du type :

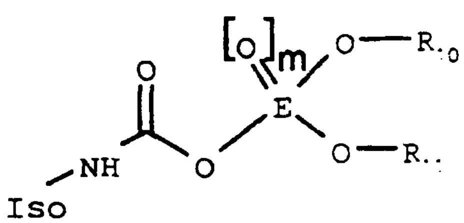

mais quand E appartient à la colonne du phosphore et que m (qui est le même que dans la formule I) est égale à zéro le composé s'isomérise (ou se fait directement) pour donner :

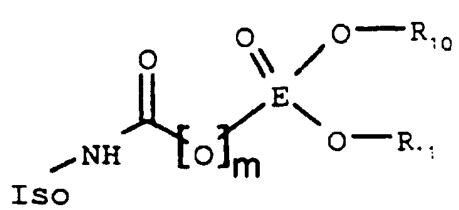

⇨ Où E est un élément de la colonne VA la classification périodique des éléments [(supplément au Bulletin de la Société Chimique de France Janvier 1966 N°1) avantageusement du phosphore]. Et donc notamment du type :

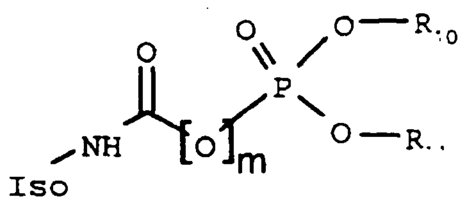

⇨ Où Iso est le reste (d'un poly)isocyanate (après élimination d'une fonction isocyanate)

⇨ où $R_{10}$ est un reste hydrocarboné (c'est-à-dire contenant des atomes d'hydrogène et de carbone) dont le point d'attache [c'est-à-dire l'atome porteur de la liaison ouverte] est un carbone

⇨ où $R_{11}$ est choisi parmi :

→ une charge négative ;

→ un groupe de formule II :

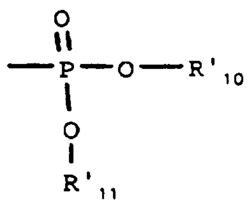

dans laquelle $R'_{10}$ est choisi parmi les restes hydrocarbonés (semblables ou différents de $R_{10}$) et une charge négative dont le point d'attache [c'est-à-dire l'atome porteur de la liaison ouverte] est un carbone
dans laquelle le(s) $R'_{11}$ est (sont) choisi(s) parmi les restes hydrocarbonés dont le point d'attache [c'est-à-dire l'atome porteur de la liaison ouverte] est un carbone (semblables ou différents de $R_{10}$ et de $R'_{11}$) et une charge négative.
Il est souhaitable qu'au moins un des substituants organiques ($R_{10}$; $R'_{11}$ ; $R'_{10}$) comporte un fragment de chaîne polyéthylène glycol avantageusement d'au moins 5, de préférence d'au moins 7 unités éthylènyloxyles. En d'autres termes il est souhaitable qu'au moins un des substituants organiques, réponde à la de même formule que les substituants de E dans la formule générale I. Plus spécifiquement au moins un des substituants organiques ($R_{10}$; $R'_{11}$; $R'_{10}$) répond à la formule

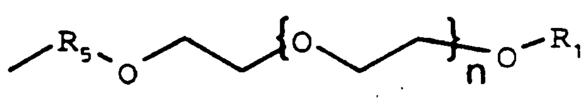

Où $R_5$ représente un bras comportant au plus deux chaînons carbonés (avec les mêmes valeurs préférées que X' et X)

où n est un entier choisi entre 0 et 30 avantageusement entre 5 et 25, de préférence entre 9 et 20 (intervalles fermés c'est à dire comprenant les bornes) ;

où $R_1$ représente un radical hydrocarboné, avantageusement choisi parmi les aryles et les alcoyles éventuellement substitués.

Ainsi selon une variante avantageuse de la présente invention, les compositions selon la présente invention présente des composés issus de la réaction exposées ci-dessus dans une proportion globale, par rapport à un volume d'un litre d'isocyanate, de 0,01 à 1, avantageusement de 0,05 à 0,5, de préférence de 0,05 à 0,3 équivalent de fonction :

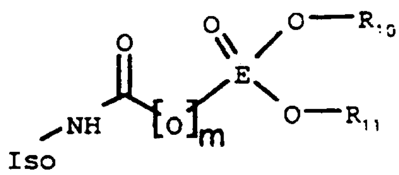

Il est avantageux que le radical Iso procure majoritairement ou en totalité un lien aliphatique avec les même préférence que celles exposées ci dessus à propos des isocyanates.

Font donc également partie de l'invention les composés de formule :

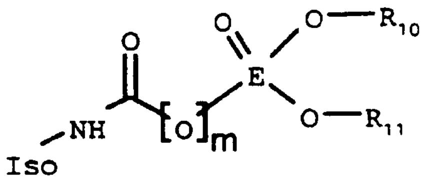

Dans laquelle R10 et R11 peuvent prendre les valeurs ci dessus mais aussi quand m est 1, être une charge négative en raison du fait que dans certain lot il peut y avoir des quantités significatives d'acide phosphorique résiduel.

Bien sur alors $R_{10}$ peut aussi valoir :

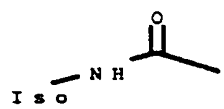

Le radical Iso pouvant ou non être alors le même que celui de l'avant dernière formule.

dans laquelle Iso représente le reste d'un polyisocyanate, avantageusement d'un produit de réaction d'un monomère diisocyanate pour former du biuret ou des isocyanurates (trimère), ou avec un di- ou poly-ol avantageusement un triol ou un tetra-ol.

Il est avantageux que le radical Iso procure majoritairement ou en totalité un lien aliphatique avec les mêmes préférences que celles exposées ci dessus à propos des isocyanates.

Avantageusement Iso porte, outre la fonction figurée sur la formule au moins une, de préférence au moins deux fonctions isocyanates, dont, de préférence, au moins une n'est pas masquée, et dont, plus préférentiellement, au moins deux ne sont pas masquées.

Un autre but de la présente invention est de fournir un procédé du type précédent qui permette de réaliser la mise en émulsion de la composition visée ci dessus lorsqu'elle ne contient pas d'eau.

Ce but et d'autres qui apparaîtront par la suite sont atteints au moyen d'un procédé de mise en émulsion qui comporte au moins l'étape suivante :

- ajout, avantageusement sous agitation très modérée, des ou de l'isocyanate dans le mélange polyol + eau.

L'agent tensioactif peut être soit dans la phase aqueuse, soit de préférence dans la phase isocyanate. Dans le premier cas les réactions entre isocyanate et ledit composé comportant une fonction anionique et avantageusement un fragment de chaîne polyéthylène glycol sont beaucoup moins importantes.

Cette agitation est de préférence manuelle ou mécanique.

Cette mise en émulsion est avantageusement menée à une température inférieure à 50°C, de préférence à l'ambiante.

Il est souhaitable de réaliser, si nécessaire, un ajustement du pH (pour atteindre une valeur avantageusement au moins égale à trois, de préférence à 4 et avantageusement au plus égal à 11 de préférence à 10 et donc avantageusement comprise entre 3 et 11, de préférence entre 4 et 10) lors de la mise en émulsion. Cet ajustement permet d'atteindre une zone avantageuse où la première (ou unique) acidité de chaque agent tensioactif selon la présente invention est neutralisée.

Selon une variante avantageuse de la présente invention, les pigments (et notamment le bioxyde de titane) sont dispersés dans le ou les polyols avant l'addition de l'isocyanate.

Un autre but de la présente invention est de fournir un procédé d'application de la composition à base d'isocyanate pour former un revêtement.

Ces buts et d'autres qui apparaîtront par la suite sont atteints au moyen d'un procédé comportant l'application d'une couche de préparation (c'est-à-dire de composition selon l'invention comportant la phase aqueuse et les constituants de la couche) dont l'épaisseur avant séchage est comprise entre 10 et 400 micromètres avantageusement entre 50 et 200 correspondant après séchage à une épaisseur comprise entre 5 et 150 micromètres, avantageusement entre 20 et 80 micromètres.

Selon une mise en oeuvre avantageuse, ce procédé comporte un séchage de 20°C à 60°C pendant une durée pouvant aller de 1/4 à 24 heures.

Avantageusement ce séchage a lieu en présence d'un solvant pour aider à l'élimination de l'eau.

Selon une mise en oeuvre particulièrement avantageuse de la présente invention, l'application est menée par pulvérisation.

La préparation des surfaces est bien connue de l'homme de métier (Par exemple phosphatations pour les composé ferreux acier ou chromatation pour les surface a base d'alumine). (on peut par exemple se référer au ouvrage suivants : "organic coating technology "volume II de H.F. PAYNE et "Paint Handbook" Edité par G.E.WEISMANTEL)

Selon la présente invention, il est ainsi possible d'obtenir des revêtements (notamment peintures ou vernis) présentant les caractéristiques techniques (ces valeur dépendent surtout des polyols utilisés) suivantes :

| mise en oeuvre et caractéristiques du revêtement<br>épaisseur sèche Iso2178 : 45 µm<br>support et traitement de celui-ci :acier traité par phosphatation : plaques R46l<br>du fournisseur Q Pannel | | |
|---|---|---|
| | propriétés minimales obtenues | usuelle |
| test din 67530 (ces valeurs n'ont d'intérêt que lorque l'on désire une peinture brillante mais non lorsque l'on veut de la peinture mat ou satinée)<br>Brillance 20°<br>60° | <br><br>0,5<br>0,5 | <br><br>80<br>90 |
| dureté König iso 1522 | 10s | 150s |
| Adhérence test din 53151 | GT-1 | GT-5 |
| Résistance au chocs test N°iso 6272<br>direct<br>inverse | 10 cm<br><br>5 cm | >100 cm<br><br>> 100 cm |
| Résistance à la méthyléthylcétone (butanone) (Double passage) | 20 | >200 |
| Tenue en extérieur QUV Din 53384 | 50 h | 800 h |

Les exemples non limitatifs suivants illustrent l'invention.

Le Rhodafac® RE610 est un mélange de mono- et de diesters phosphorique selon la formule II, la formule moyenne de son radical hydrocarboné est un nonylphénol polyéthoxylé (~dix fois). le rapport molaire entre mono ester et diester est de environ 1 (arrondi mathématique). De même le Rhodafac® PA17 présente comme produit selon la présente invention un mélange de mono- et de diesters phosphorique selon la formule II, la formule moyenne de son radical hydrocarboné est un nonylphénol polyéthoxylé (~cinq-six fois).

<u>**Exemple 1**</u> - préparation du mélange 1

on mélange 165g de tolonate® HDT (oligomère isocyanurate à base de trimère) avec 24g d'acétate de butyle et 13g de Rhodafac® RE610 (mélange de mono- et de diesters phosphorique selon la formule II)et 3g de triéthylamine. Ce mélange est agité à l'aide d'une pale cadre ou défloculeuse pendant 5 minutes à 100 tours/minute. Ce mélange a une viscosité de 0,84 Pa.s. A 20°C et une coloration inférieure à 100 APHA.

<u>**Exemple 2**</u> - préparation du mélange 2

le mélange a la même composition que le mélange 1 mais le tolonate® HDT est remplacé par le tolonate® HDT-LV.

La viscosité de ce mélange est de 0,476Pa.s. A 20°C et une coloration inférieure à 100 APHA.

**Exemple 3** - préparation du mélange 3

le mélange est fait avec 92g de tolonate® HDT et 10g de Rhodafac RE610 et 2,3g de triéthylamine. La viscosité de ce mélange est de 5,2 Pa.s. A 20°C et la coloration inférieure à 100 APHA.

**Exemple 4 -**

30g du mélange 1 est ajouté à 20g d'eau. Le mélange est agité à l'aide d'une pale cadre à 250 tours/min pendant 5 minutes. On obtient ainsi une émulsion dont la granulométrie moyenne est 1,2 µm. La taille des gouttes d'émulsion reste stable pendant 30 heures à un pH situé entre 3 et 9.

**Exemple 5**

30g du mélange 3 est ajouté à 20g d'eau. L'émulsion est préparée dans les mêmes conditions que dans l'exemple 4. La taille moyenne des gouttes est de 1,1 µm.

**Exemple 6 comparatif**

30g d'un mélange HDT-LV / acétate de butyle (même rapport pondéral que dans l'exemple 2) est ajouté à 20 g d'eau. L'émulsion est préparée dans les mêmes conditions que dans l'exemple 4. On obtient ainsi une émulsion grossière dont la taille moyenne est supérieure à 5µm et donc difficile à caractériser.

**Exemple 7 (comparatif)**

On mélange 165g de tolonate® HDT avec 13 g de Rhodocal® AT60 (dodecylbenzène sulfonate de triéthylamine). Ce mélange est agité à l'aide d'une pale cadre à 100 tours/min. Pendant 5 minutes. 10g de ce mélange est ajouté à 90 g d'eau sous agitation à l'aide d'une pale cadre à 400 tours/min. Pendant 10 minutes. Ensuite le produit est analysé, il est constitué de deux phases, l'une riche en huile et l'autre riche en eau mais il n'y a pas formation d'une émulsion homogène.

**Exemple 8**

On mélange 83 g de tolonate® HDB (biuret) avec 6g de Rhodafac® PA17 et 1,2g de triéthylamine et 10g d'acétate de butyle. Ce mélange est homogénéisé à l'aide d'une pale cadre à 100 tours/min. Pendant 5 minutes.

10 g de ce mélange est ajouté à 90 g d'eau sous agitation à l'aide d'une pale cadre à 200 tours/min. Pendant 5 minutes. L'émulsion obtenue présente une granulométrie centrée autour de 3,5µm.

**Exemple 9**

on mélange 83g de tolonate® HDT avec 6g de Rhodafac® PA17 et 1,2g de triéthylamine et 10g d'acétate de butyle. Ce mélange est homogénéisé à l'aide d'une pale cadre à 100 tours/min. Pendant 5 minutes.

10 g de ce mélange est ajouté à 90 g d'eau sous agitation à l'aide d'une pale cadre à 200 tours/min. Pendant 5 minutes. L'émulsion obtenue présente une granulométrie centrée autour de 0,98µm.

**Exemple 10-** Préparation d'un vernis à partir du mélange 1 et d'un polyol commercialisé par la société SC JOHNSON POLYMER sous le nom de JONCRYL® 540.

Ce polyol est une émulsion de copolymères acryliques d'extrait sec 42% et d'indice d'hydroxyle 42 (sur matière sèche).

On prépare un vernis par incorporation de 7,3 g du mélange 1 dans 92,7 g de JONCRYL® 540. L'incorporation se fait sous agitation manuelle avec une spatule pendant 10 minutes. Les proportions entre le mélange 1 et le polyol sont telles que le rapport molaire NCO/OH soit égal à 1/1.

Des films de vernis ont été appliqués avec une tige filetée (de façon à avoir une épaisseur sèche de 40µm) 1/2 heure et 4 heures après l'incorporation de l'isocyanate dans le polyol.

Les performances obtenues à 1/2 heure et 4 heures de vieillissement du mélange, après 48 heures de séchage à 23°C et 55% d'humidité relative, sont équivalentes et d'un bon niveau : brillance de 89 sous un angle de 20°, résistance à la Méthyl Ethyl Cétone supérieure à 100 aller-retour avec un coton imbibé, dureté pendulaire Persoz égale à 140 s.

**Exemple 11 -** Préparation d'une peinture blanche brillante à partir du mélange 2 et d'un polyol

Une peinture sera constituée de 2 composants : un élément A contenant notamment et le plus souvent majoritairement, le pigment et le polyol, et un élément B constitué uniquement du mélange 2.
Le polyol utilisé dans le présent exemple est celui commercialisé par la société JAGER sous le nom de JAGOTEX® F 313.
Ce polyol est une solution aqueuse d'une résine de copolymères acryliques contenant des groupes acides et hydroxyles. Il est neutralisé avec de la Di Méthyl Ethyl Amine à un pH de 8,5, il a un extrait sec de 45%, un indice d'acide de 60 et un indice d'hydroxyle de 132.
100g d'élément A est obtenus par mélange sous agitation poussée (20 minutes à 1000 tours/mn avec une pale défloculeuse) de 49,7 g de JAGOTEX® F 313, de 0,8 g de DISPERBYK® 181 (agent mouillant commercialisé par la société BYK), de 0,3 g de BYK® 022 (agent antimousse commercialisé par la société BYK), de 23,7 g de TITAFRANCE® RL 60 (pigment de bioxyde de titane commercialisé par la société RHONE-POULENC) et de 25,5 g d'eau déminéralisée.
Une peinture est préparée par incorporation sous agitation réduite (environ 300 tours/mn avec pale défloculeuse) de 3,9 g d'élément B (mélange 2) dans le composant A. Ces proportions sont telles que le rapport Pigment/Liant de la peinture soit égal à 0,75/1 et que le rapport NCO/OH dans la peinture soit égal à 1/1.
Des films de peinture ont été appliqués sur des plaques d'acier avec une tige filetée (de façon à avoir une épaisseur sèche de 30μm) 1/2 heure après l'incorporation de l'isocyanate dans le polyol. La durée d'utilisation de la peinture (mélange A + B) est de 3 heures.
Les performances des films, après 48 heures de séchage à 23°C et 55% d'humidité relative, sont d'un bon niveau : brillance de 71 sous un angle de 20° et 84 sous un angle de 60°, résistance à la Méthyl Ethyl Cétone supérieure à 100 aller-retour avec un coton imbibé, dureté pendulaire Persoz égale à 100 s, temps de séchage "sec au toucher" égal à environ 3 heures à 23°C et 55 % d'humidité relative.
**Exemple 12** - Préparation d'une peinture blanche à partir du mélange de l'exemple 6 et du polyol JAGOTEX® F 313 commercialisé par la société JAGER (cf. Caractéristiques données dans l'exemple 12).
La peinture sera obtenue par mélange d'un élément A contenant le pigment et le polyol, et d'un élément B constitué uniquement du mélange de l'exemple 6.
L'élément A est préparé selon le procédé et la formule de l'exemple 11.
La peinture est préparée par incorporation sous agitation réduite (environ 300 tours/mn avec pale défloculeuse) de 3,6 g d'élément B (mélange de l'exemple 6) dans le composant A. Ces proportions sont telles que le rapport Pigment/Liant de la peinture soit égal à 0,75/1 et que le rapport NCO/OH dans la peinture soit égal à 1/1 (conditions identiques à celles de l'exemple 11).
Des films de peinture ont été appliqués dans les mêmes conditions que celles de l'exemple 11
Les performances des films, après 48 heures de séchage à 23°C et 55% d'humidité relative, sont médiocres : brillance de 8 sous un angle de 20° et 27 sous un angle de 60° et résistance à la Méthyl Ethyl Cétone inférieure à 10 aller-retour avec un coton imbibé.
La comparaison de cet exemple et de l'exemple 11 montre la potentialisation apportée par la combinaison avec tensioactif Rhodafac RE 610 notamment neutralisé avec la triéthylamine.

**Exemple 13** - utilisation de Nanolatex;

Préparation d'un vernis à partir du mélange 1 et d'un nanolatex-c'est-à-dire latex dont la taille particulaire est Nanométrique plus précisément dont le $d_{50}$ est au plus égal à environ 100 nanomètres]) de monomères acryliques.
Le nanolatex utilisé est un produit expérimental réalisé selon le procédé décrit dans la demande de brevet d'invention français déposé le 28 avril 1995 N° 95/05123 et dans la demande réflexe de brevet Européen N°EP 0.739.961 de caractéristiques suivantes :

$d_{50}$ : environ 35 nm
fonction carboxylate : 2,6 % en poids sur le polymère sec
fonction ol : 2,6 % en poids sur le polymère sec
Masse moléculaire supérieure à 100 000
Extrait sec en poids : 30 %
pH environ 8
Température Minimum de Formation de Film environ 20°C
Température de transition vitreuse environ 40°C

Le vernis est préparé par incorporation avec mélange manuel de 4, 6 g du mélange 1 dans 45,6 g de nanolatex.

Ce ratio correspond à un rapport des fonctions NCO/OH de 1,2.

Le mélange ainsi préparé présente une durée de vie de 4 heures, ceci signifie que pendant 4 heures la viscosité et l'aspect du mélange est inchangé mais aussi que les films formés à partir de ce mélange pendant ces 4 heures ont des propriétés comme la résistance au solvant, la dureté et la brillance inchangés.

Le temps de séchage du film est remarquablement court : 20 minutes pour le temps "hors poussière" et 30 minutes pour le temps "sec au toucher" selon la norme NF T 30037. Ces mesures ont été faites sur plaques de verre pour une épaisseur sèche de film de 40 µm et un séchage à 23°C avec 55% d'humidité relative.

Le fait d'obtenir un temps de séchage court et un durée de vie du mélange relativement long constitue un avantage très recherché par les experts en peinture.

Les valeurs d'usage du revêtement sont également globalement d'un très bon niveau, on peut citer:

- la dureté Persoz du film de 50 µm sec mesurée sur plaque d'acier après séchage de 24 heures à 23°C et 55 % d'humidité relative est égale à 275 secondes.
- la brillance sous un angle de 20° du film appliqué sur plaque d'acier est égale à 90
- la résistance à la Méthyl Ethyl Cétone du film séché pendant 7 jours à 23°C et 55 % d'humidité relative est supérieure à 200 aller-retour avec un coton imbibé.

Ces excellentes performances de cette association Nanolatex-polyisocyanate selon l'invention sont certainement imputables à la physico-chimie très particulière de ces produits notamment à la très importante surface spécifique du nanolatex qui favorise une grande homogénéité de réticulation dans le polymère final.

**Exemple 14**

Le mélange préparé dans l'exemple 13 est appliqué à la brosse avec un dépôt de 200 g/m2 sur un support de type béton.

Après séchage de 1h à température ambiante, le revêtement offre une hydrofugation du support qui se traduit par un effet perlant, et une moindre absorption d'eau. De plus le revêtement présente une bonne résistance à l'abrasion.

**Exemple 15 -**

Préparation d'un revêtement à partir du mélange 1 et d'un nanolatex de monomères acryliques.

Le nanolatex est un produit expérimental selon la demande d'invention N° de caractéristique suivante :

$d_{50}$ environ 35 nm
fonction carboxylate : 1 % en poids sur le polymère sec
fonction ol : 2,6 % en poids sur le polymère sec
Masse moléculaire supérieure à 100 000
Extrait sec en poids : 30 %
pH environ 8
Température de transition vitreuse environ - 30°C

Le vernis est préparé par incorporation avec mélange manuel de 4, 6 g du mélange 1 dans 45,6 g de nanolatex. Ce ratio correspond à un rapport des fonctions NCO/OH de 1,2.

Le revêtement est appliqué sur béton à la brosse à raison de 2 couches de 300 g/m2. Sa grande souplesse, générée principalement par le faible Tg du nanolatex, lui confère une capacité à masquer les fissure pouvant apparaître lors du vieillissement du support (notamment à cause de la dilatation du béton à cause de grande variation de température).

De plus la réticulation avec le polyisocyanate selon l'invention confère au revêtement une excellente résistance aux agents chimiques et à l'eau.

**Revendications**

1. Composition pour addition successive ou simultanée comprenant :

   - un composé (poly)isocyanate non masqué ou partiellement masqué, dans lequel une des fonctions NCO est reliée à un squelette hydrocarboné, par l'intermédiaire d'un carbone saturé ($sp^3$) ; et
   - un agent tensioactif contenant au moins un composé comportant une fonction anionique et avantageusement

un fragment de chaîne polyéthylèneglycol d'au moins 1, de préférence d'au moins 5, unités éthylèneoxyle.

**2.** Composition selon la revendication 1, **caractérisée en ce que** ledit composé comprenant une fonction anionique comporte une partie hydrophile formée de ladite fonction anionique dudit fragment de chaîne polyéthylèneglycol et d'une partie lipophile à base d'un radical hydrocarboné.

**3.** Composition selon la revendication 1 ou la revendication 2, **caractérisée par le fait que** ladite partie lipophile est choisie parmi les groupes alcoyle et aryle.

**4.** Composition selon l'une des revendications 1 à 3, comprenant en outre une phase aqueuse.

**5.** Composition selon l'une quelconque des revendications 1 à 4, **caractérisée en ce que** le composé (poly)isocyanate contient des structures de type isocyanurate, des structures urétidine dione, des structures biurets, des structures allophanates, ou une combinaison de ce type de structure sur une seule molécule ou en mélange.

**6.** Composition selon la revendication 5 dans laquelle le (poly)isocyanate est un mélange d'hexaméthylène diisocyanate contenant des structures de type isocyanurate (HDT), et d'isophorone diisocyanate contenant des structures de type isocyanurate (IPDT).

**7.** Emulsion ou suspension aqueuse comprenant au moins une composition selon l'une quelconque des revendications 1 à 6.

**8.** Composition ou émulsion selon l'une quelconque des revendications 1 à 7 comprenant en outre un composé à hydrogènes réactifs.

**9.** Composition ou émulsion selon la revendication 8 dans laquelle le composé à hydrogènes réactifs est un polyol.

**10.** Composition ou émulsion selon la revendication 9 dans laquelle le rapport molaire entre les fonctions isocyanates libres et les fonctions hydroxyles est compris entre 0,5 et 2,5, avantageusement entre 0,8 et 1,6, avantageusement entre 1 et 1,4.

**11.** Procédé d'application d'une composition ou d'une émulsion selon l'une des revendications 1 à 10, comportant l'application d'une couche de ladite composition ou de ladite émulsion dont l'épaisseur avant séchage est comprise entre 10 micromètres et 400 micromètres, avantageusement entre 50 et 200 micromètres,
pour la formation d'un revêtement.

**12.** Procédé d'application selon la revendication 11 comportant une étape ultérieure de séchage de 20°C à 60°C pendant une durée pouvant aller de 1/4 à 24 heures.

**13.** Revêtement obtenu selon le procédé de la revendication 11 ou de la revendication 12.

**14.** Revêtement selon la revendication 13, **caractérisé en ce qu'**il présente, après séchage, une épaisseur comprise entre 5 micromètres et 150 micromètres, avantageusement entre 20 micromètres 80 micromètres.

**15.** Peinture ou vernis à base d'au moins un revêtement selon la revendication 13 ou selon la revendication 14.